# EUROPEAN PATENT SPECIFICATION

(11) **EP 1 856 520 B1**
(45) Date of publication and mention of the grant of the patent: **17.06.2015**
(21) Application number: 06717254.4
(22) Date of filing: 03.01.2006
(51) Int. Cl.: G01N 33/543, G01N 33/48

(54) **MICROWAVE ACCELERATED PLASMONICS**
MIKROWELLENBESCHLEUNIGTE PLASMONICS
PROCEDES ET SYSTEMES PLASMONIQUES D'ACCELERATION A MICRO-ONDES

(30) Priority: 03.01.2005 US 641245 P
(43) Date of publication of application: 21.11.2007
(73) Proprietor: UNIVERSITY OF MARYLAND BALTIMORE COUNTY, Baltimore, MD 21250 (US)
(72) Inventor: GEDDES, Chris D., Bel-Air, MD 21015 (US)
(74) Representative: Chalk, Anthony John
(86) International application number: PCT/US2006/000029
(87) International publication number: WO 2006/074130

(56) References cited:
- WO-A1-02/14868
- WO-A2-2006/137945
- HIRSCH L R ET AL: "A WHOLE BLOOD IMMUNOASSAY USING GOLD NANOSHELLS" ANALYTICAL CHEMISTRY, AMERICAN CHEMICAL SOCIETY, US, vol. 75, no. 10, 15 May 2003 (2003-05-15), pages 2377-2381, XP001170909 ISSN: 0003-2700
- BUDDE U ET AL: "DRASTISCHE VERKUERZUNG VON INKUBATIONSZEITEN BEI IMMUNHAEMATOLOGISCHEN UNTERSUCHUNGEN DURCH EINSATZ VON MIKROWELLENGERAETEN" INFUSIONSTHERAPIE UND TRANSFUSIONSMEDIZIN, BASEL, CH, vol. 22, no. SUPPL. 01, 1 January 1995 (1995-01-01), pages 92-94, XP000985251 ISSN: 1019-8466
- ELGHANIAN ROBERT ET AL: "Selective colorimetric detection of polynucleotides based on the distance-dependent optical properties of gold nanoparticles" SCIENCE (WASHINGTON D C), vol. 277, no. 5329, 1997, pages 1078-1081, XP002566839 ISSN: 0036-8075
- ASLAN ET AL: "Microwave-Accelerated Surface Plasmon-Coupled Directional Luminescence: Application to fast and sensitive assays in buffer, human serum and whole blood" JOURNAL OF IMMUNOLOGICAL METHODS, ELSEVIER SCIENCE PUBLISHERS B.V.,AMSTERDAM, NL, vol. 323, no. 1, 11 May 2007 (2007-05-11), pages 55-64, XP022071916 ISSN: 0022-1759
- KOGAN M.J. ET AL.: 'Nanoparticle-Mediated Local and Remote Manipulation of Protein Aggregation' NANO LETTERS vol. 6, no. 1, 2006, pages 110 - 115, XP003012734
- ZHAO Y. ET AL.: 'Microwave-Induced Polyol-Process Synthesis of Copper and Copper Oxide Nanocrystals with Controllable Morphology' EUR. INORG. CHEM. vol. 20, 2004, pages 4072 - 4080, XP003012735
- MA ET AL. ANAL. BIOANAL. CHEM. vol. 382, 2005, pages 1044 - 1048, XP019327424
- ZHANG J. ET AL.: 'Complexation of polysaccharide and monosaccharide with thiolate boronic acid capped on silver nanoparticle' ANALYTICAL BIOCHEMISTRY vol. 332, 2004, pages 253 - 260, XP003012736

## Description

### BACKGROUND OF THE INVENTION

### Technical Field

The present invention is directed to a system and method for increasing sensitivity of a detection system, and more particularly, to the use of low power microwaves for speeding up reaction kinetics for synthesizing nanostructures for use in glucose testing and/or a plasmonic detection system.

### Background of Related Art

Over the past 10 years, fluorescence has become a dominant technology in medical testing, drug discovery, biotechnology and cellular imaging. The use of fluorescence technology has greatly enhanced the ability to detect specific molecules, leading to rapid advancements in diagnostics. For example, fluorescence detection is widely used in medical testing and glucose analysis because of the high degree of sensitivity obtained using fluorescent techniques. Small numbers of molecules can be detected using fluorescence technology.

The present inventor, in addition to his colleagues, discovered that close-proximity metallic silver islands or colloids can alter the radioactive decay rate and/or excitation rate of fluorophores. Further, it has been shown that quantum yield of low quantum yield fluorophores can be increased by proximity to metallic surfaces. The enhanced excitation of fluorophores in close proximity to metallic surfaces including islands, colloids, porous and continuous surfaces can have numerous applications in the biochemical and biological applications of fluorescence because of the increased intensity of the fluorescence.

For example, surface-based assays, in which the amount of target is quantified by capturing it on a solid support and then labeling it with a detectable label, are especially important since they allow for the facile separation of bound and unbound labels. Often detection of binding complexes in array-based assays involves the detection of a fluorescently labeled species that is part of the binding complex. Optical glucose monitoring is one example of an extremely important and active field of research. The goal of this research is to provide a noninvasive method of monitoring and more optimally managing diabetes, a disease that affects millions of people worldwide. A variety of approaches are currently being pursued, including near-and mid-infrared spectroscopy, photoacoustic spectroscopy, polarimetry, diffuse light scattering, and Raman spectroscopy (Waynant, R. W. et al, IEEE-LEOS Newsletter 12:3-6 (1998)) and plasmonic based sensing systems using surface plasmons.

Hirsch R.W. et al (Anal. Chem. Vol. 75, No. 10, 15 May 2003, p 2377-2381) teach the use of gold/silica nanoshells in immunoassays but are not concerned with the use of microwave energy to increase the reaction time of the system. Budde U. et al (Infusionstherapie und Transfusionsmedizin, Vol. 22, No. Suppl. 01, 1 January 1995, p 92-94) disclose the use of microwave energy in enzyme-linked agglutination immunioassays, but are concerned with a system of Budde using plastic vials, so do not consider the use of low power energy. Elghanian R. et al (Science, Vol. 277, No. 5329, 1997, p 1078-1081) discuss immunoassays that require the use of 13 nm solid gold balls having attached thereto different nucleotides that interact with each other, so that the aggregation of the derived complexes cause a colour change. However, these authors do not consider the use of microwave energy with these gold nanoparticles.

WO-A-02/14868 describes a method for carrying out enzyme-linked immunosorbent assay for detection of minute quantities of biomolecules such as antigens or antibodies and relates to microwave mediated immobilization of biomoleules on an activated surface followed by subsequent controlled microwave irradiation. However, the document again fails to consider the use of microwave radiation with metallic particles. WO-A-2006/137945 addresses increasing fluorescence detection in surface assay systems while increasing kinetics of a bioreaction therein by providing low-power microwaves to irradiate metallic materials within the system in an amount sufficient to increase heat, thereby affecting the kinetics of a bioreaction therein. However, there is no consideration of the use of a complexing agent that results in the aggregation of the metallic particles.

Surface plasmons are electron oscillations on the surface of metals. However, these plasmons are usually non-radiative and difficult to put to practical use. Recently it has been discovered by the present inventor that surface plasmons are easily generated and manipulated using the appropriate metal structures, such as metal films or metallic nanostructures of appropriate size and shape. Metal nanostructures have been studied extensively and are emerging as important colorimetric reporters due to their high extinction coefficients, which are typically several orders of magnitude larger than those of organic dyes. In particular, nanostructures made from the noble metals, such as those of silver or gold, with their associated strong plasmon resonance, have generated great interest. Notably, nanostructure aggregation results in further color changes of nanostructure solutions due to mutually induced dipoles that depend on interparticle distance and aggregate size.

However, effective aggregation of applicable metallic nanostructures for determinative color changes is limited by the binding and chemical reactions between the metallic nanostructures and aggregating compounds that bind thereto. Thus, it would be beneficial to increase the kinetics of the metallic nanostructures and aggregating compounds without damaging the participating compounds.

### SUMMARY OF THE INVENTION

The fact that the plasmon resonance is a sensitive function of nanostructure geometry, coupled with synthetic advances that allow for the controlled and systematic variations in nanostructure geometry, is leading to the expansion of the sensing field called "plasmonics". Gold and/or silver nanoparticle aggregation induced by analytes has been demonstrated to be effective for DNA, metal ions and antibodies, but heretofore has not been used for glucose sensing.

The present invention relates to increasing the aggregation rate of the metallic nanostructures with non-metallic compounds that are complexed in the aggregation process.

Thus, the present invention provides a detection method comprising:
providing metallic nanoparticles wherein the metallic nanoparticles are modified with multiple immobilized groups having affinity for a complexing agent;
introducing the complexing agent for binding with immobilized groups;
applying microwave energy having a frequency range of from 10⁸ to 10¹² Hz to cause an increase in heat in the system and/or increase the kinetics of a chemical reaction between the immobilized group and complexing agent within the detection system thereby increasing aggregation of the metallic particles when the complexing agent and immobilized group react, wherein the microwave energy has an intensity in a range of from about 0.0001 µW/cm² to about 1000 µW/cm²; and
measuring the change in plasmonic absorption due to aggregation.

Preferably, the metallic nanostructure comprises dextran immobilized on gold nanoparticles that are combined with concanavalin A (Con A from Canavalia ensiformis), to form the aggregates.

The method of the present invention may be further used in increasing the competitive binding of glucose in a detection system, wherein the detection system comprises a metallic nanostructure aggregate and with the addition of glucose the competitive binding of glucose causes a change in monitored absorption, and wherein applying low-power microwave energy to the system reduces the time period required for such competitive binding and subsequent change in absorption.

Additional aspects, features and embodiments of the invention will be more fully apparent from the ensuing disclosure and appended claims.

### BRIEF DESCRIPTION OF THE DRAWINGS

FIG. 1 is a schematic representation of a model protein system demonstrating microwave-accelerated plasmonics, using biotinylated-BSA coated 20 nm gold (Au) colloids cross-linked by streptavidin.
FIG. 2 shows absorption spectra of 20 nm gold colloids as a function of concentration, before and after exposure to low power microwaves.
FIG. 3 shows the change in absorbance at 600 nm as a function of time for biotinylated -BSA coated 20 nm gold colloids crosslinked by streptavidin.
FIG. 4 shows the change in absorbance of biotinylated-BSA 20 nm gold colloids crosslinked by different additions of streptavidin, both without (20 min incubation) and after low power microwave heating, with FIG. 4A showing the effect of a 5 nM streptavidin addition, FIG. 4B showing the effect of a 10 nM streptavidin addition, FIG. 4C showing the effect of a 20 nM streptavidin addition, and FIG. 4D showing the change in absorbance at 650 nm for both the room temperature incubated and microwave heated samples.
FIG. 5 shows absorption spectra of biotinylated-BSA 20 nm gold colloids in the presence of blocked streptavidin, after both a 30 minute incubation period and 10 seconds microwave heating.
FIG. 6 shows the change in absorbance of both 20nm (FIG. 6A) and 200 nm (FIG. 6B) virgin gold colloids as a function of temperature, and the respective change in absorbance as a function of temperature (FIG. 6C).
FIG. 7 shows absorption spectrum of thymol blue as a function of temperature (FIG.7A), the same solution containing 20 nm colloids (FIG. 7B), and the respective A600 / A425 ratiometric plots Vs temperature (FIG. 7C).
FIG. 8 shows absorbance spectra of 450 µL of 20 nm gold colloids and thymol blue microwave heated in the black body for different times (FIG. 8A), 450 µL of thymol blue solely heated in the black body (FIG. 8B), and the respective ratiometric plots (FIG. 8C).
FIG. 9 shows absorbance spectra of 20 nm gold colloids in pH 6.0, 9.0 and after microwave heating.

### DETAILED DESCRIPTION OF THE INVENTION

The present invention relates to microwave-accelerated plasmonics, which utilizes low-level microwaves to markedly enhance the speed of plasmonic changes that are used for detection, thereby dramatically expanding the usefulness of plasmonic detection for a wide spectrum of sensing, monitoring and other applications.

Specific applications of the invention include solutions and assays that employ metal colloids and changes in aggregation of such colloids due to addition of a test sample, e.g., matter containing or susceptible of containing an analyte of interest. By introducing low-level microwave energy to the testing system, the aggregative changes occur more rapidly, as compared to a corresponding system in which such microwave energy is not added. Such aggregative changes may involve increases, or alternatively decreases, in aggregation, depending on the specific system involved, and changes in plasmonic absorption due to changes in the aggregation are readily measured, e.g., to provide output indicative of the presence and/or concentration of a target or agent of interest.

As a consequence of heating by low-level microwaves, changes in aggregation take place in a significantly reduced time-frame, so that the plasmon absorption profile correspondingly undergoes fast change, to facilitate the detection process much more rapidly than has heretofore been possible.

The present invention in various applications employs low power microwaves for heating of samples to greatly speed up biological/chemical kinetics within such samples. Low power microwaves do not destroy or denature proteins, DNA, or RNA, but equally heat the sample all over, providing for accelerated kinetics in applications such as binding or hybridization.

In practice any microwave energy source may be used. Preferably, the source is a magnetron generating microwave energy that is transported as an electromagnetic wave in a frequency range between about 10⁸ to about 10¹² Hz. A feed structure may be included that guides the microwaves from the energy source to a containment chamber containing the detection assay or system. Electronic controls are usefully employed to control the microwave energy source and the power of said energy. Preferably, the power is maintained between about 0.0001 *µW*/cm² to about 1000 *µW*/cm² and more preferably from about 0.0001*µW*/cm² to about 0.01*µW*/cm².

A typical electromagnetic processing system operating at microwave frequencies includes several related components: (1) an energy source, usually a constant frequency microwave oscillator, (2) transmission lines, e.g., a waveguide or coaxial cable, (3) an applicator and containment chamber, and (4) the process material itself. The containment chamber preferably is formed of microwave reflective material and is designed to prevent leakage of microwave energy to the environment outside the containment chamber.

Additionally, maser technology may be used in the practice of the present invention. Masers are devices that amplify or generate electromagnetic energy waves with great stability and accuracy. Masers operate on the same principal as lasers, but produce electromagnetic energy in the radio and microwave, rather than visible range of the spectrum. In masers, the electromagnetic energy is produced by the transition of molecules between rotational energy levels.

In one embodiment of the invention, low-power microwave energy is used in the synthesis of dextran-coated gold colloids and subsequent aggregation with the addition of Con A, thereby providing for useful sensing aggregates, which show plasmon changes in the presence of glucose which is widely known to competitively bind Con A. Further, by altering the gold colloid size, the dextran molecular weight and the concentration of Con A used to form the sensing aggregate, dynamic glucose sensing range can be fine-tuned.

The dextran-coated gold colloids, which have been aggregated by the controlled addition of Con A can be synthesized with enhancement of the synthesizing regime by applying low power microwaves to increase the kinetics of chemical reactions in the aggregation process.

The invention thus relates to microwave-accelerated plasmonics, which in specific embodiments utilizes low power microwaves to kinetically accelerate assays which use plasmon resonance particles.

The plasmon resonance particles utilized in the plasmonics detection method of the invention can be formed of any suitable material, such as for example gold, copper, silver, aluminum, and alloys including one or more of such metals.

The plasmon resonance particles can for example be used to sense analytes, e.g., in clinical, industrial or environmental applications. As another example, such plasmon resonance particles can be employed for imaging applications, such as in the detection of cancers, genomic abnormalities etc.

In use, the plasmon resonance particles can be used for sensing in different ways, such as for example colorimetrically, by changes in their absorption based properties, in exposure to light, and/or by changes in scattering properties of the plasmon resonance particles, in exposure to white light or monochromatic/laser light.

The plasmon resonance particles can change these properties in response to their close proximity, a function of particle aggregation, since the plasmon resonances will interact at distances of approximately 2.5 times the diameter of the plasmon resonance particles. The plasmon resonance particles also exhibit changed properties in response to long range coupling at distances of 2.5 times their radius, so that large colloids can be used to couple over larger distances. The plasmon resonance particles also change properties in response to changes in local particle refractive index.

The microwave-accelerated plasmonic process of the invention is usefully applied to a wide variety of solution based plasmon resonance particle assays. A broad spectrum of proteins, DNA and RNA can be used to aggregate plasmon resonance particles using microwave heating, and thereby can be sensed by this rapid diagnostic approach.

Although plasmon absorption and plasmon light scattering have been used in assays for detection, imaging and biosensing, no one has considered accelerating plasmonic kinetics using low power microwaves. The present invention therefore achieves a major advance in the art, and enables new detection, assay and imaging products, including, without limitation: microwave-accelerated plasmon absorption based detection for drug screening; plasmonic scattering assays; plasmon scattering based imaging, e.g., in cancer expression and determination applications; rapid detection of DNA targets, for clinical or bioterrorism-related applications; rapid detection of RNA targets, such as for rapid avian bird flu detection; and rapid detection of protein-based targets for clinical, pharmaceutical and research applications.

The microwave-accelerated plasmonics process of the invention is usefully employed to facilitate the rapid detection of any analyte that causes a change in either the plasmon resonance absorption band (colorimetrically) or by rapid changes in the light scattering properties of the particles.

The invention reflects the surprising and unexpected finding that low power microwaves, e.g., in a range of about 0.0001 µW/cm² to about 1000 µW/cm², do not perturb plasmon resonances of metallic nanostructures, a discovery that is at odds with the conventional experience of metallic structures being imcompatible with microwave exposure. It is common experience that metallic structures in microwave cavities typically cause arcing or sparking, such as when a teaspoon is inadvertently left in a microwave oven that is subsequently turned on.

For metals, attenuation of microwave radiation arises from the creation of currents resulting from charge carriers being displaced by the electric field. These conductance electrons are extremely mobile and unlike water molecules can be completely polarized in 10⁻¹⁸ s. In microwave cavities employed in the practice of the invention, the time required for the applied electric field to be reversed is far longer than this, in fact many orders of magnitude. If metal particles are large, or form continuous strips, then large potential differences can result, which can produce dramatic discharges if they are large enough to break down the electric resistance of the medium separating such large metal particles.

Interestingly, I have discovered that small metal particles do not generate sufficiently large potential differences for such arcing and sparking phenomena to occur, but the charge carriers that are displaced by the electric field are subject to resistance in the medium in which they travel due to collisions with the lattice phonons. This in turn leads to ohmic heating of the small metal particles in addition to the heating of any surface polar molecules, e.g., in an associated solvent medium. Thus, assays and other detection applications are enabled, in which such localized heating rapidly accelerates the kinetics of the assay or other detection process.

Thus, there is provided a method of increasing sensitivity of a detection or imaging system adapted to detect or image a target in a sample by change in plasnionic resonance of particles in interaction with the target, in which the method includes introducing microwaves to the sample that accelerate such interaction. The interaction may involve aggregation of the particles and target. The target may include any of proteins, DNA and RNA or other target materials, agents or species. The particles can include metal selected from among gold, copper, silver, aluminum, and alloys including one or more of said metals. The method can further include the step of detecting the presence or concentration of the target in the sample by the change in plasmonic resonance of said particles, wherein such change comprises a colorimetric change, or alternatively a change in the scattering properties of the particles in exposure to light, e.g., white light, monochromatic light, laser radiation, etc. Preferably, the microwaves are non-perturbing of the plasmonic resonance, and non-degradative of the target and sample.

The microwaves used in the method of the invention have frequency in a range of from about 10⁸ to about 10¹² Hz and the intensity of the microwave radiation is advantageously in a range of from about 0.0001 µW/cm² to about 0.01 µW/cm².

In one specific embodiment, the method of the invention is adapted for RNA target detection for determination of presence of avian influenza in a biological sample, e.g., from a human or other animal. In another specific embodiment, the method of the invention is adapted for glucose monitoring.

The present invention in another aspect contemplates a plasmonic resonance detection or imaging system arranged for detecting or imaging a target in a sample by change in plasmonic resonance of particles in interaction with the target, in which the system includes a microwave source arranged to introduce microwaves to the sample that accelerate such interaction. The microwave source in such system can comprise a constant frequency microwave oscillator, and a waveguide adapted to transmit microwaves from the oscillator to the sample.

The features and advantages of the invention are more fully shown in reference to the following non-limiting illustrative examples.

### EXAMPLE 1

### Materials

Gold nanoparticle dispersions (monodisperse, either 20 or 10 nm average particle diameter), concanavalin A (Con A from Canavalia ensiformis), dextran (average molecular weight: 64000 and 505000) hydrogen peroxide, sulfuric acid, sodium phosphate monobasic, phosphate-buffered saline (PBS), absolute ethanol, 2-(2-aminoethoxy)ethanol (AEE) and N-hydroxy-2,5-pyrrolidinedione (NHS) were obtained from Sigma. 16-Mercaptohexadecanoic acid (16-MHDA) and polyoxyethylene (20) sorbitan monolaurate (Tween 20), epichlorohydrin, 2-methoxyethyl ether (diglyme), and nitric acid were obtained from Aldrich. N-3-(Dimethylaminopropyl)-N'-ethyl-carbodiimide (EDC) was obtained from Fluka. All chemicals were used as received.

### Buffers and solutions

Sodium phosphate monobasic buffer solution was prepared to a 10 mM concentration at pH 7. PBS was dissolved in deionized water and the pH was adjusted to 7.4. Exact pH values for buffer solutions were obtained using a Beckman pH meter. Deionized water (>18 MΩ/cm) was used in the preparation of all buffer solutions. All glassware was washed with "piranha solution" (3:7, 30% H₂O₂/H₂SO₄) prior to use. Solutions of 0.50 mM 16-MHDA were prepared in degassed ethanol. Tween 20 solutions were prepared in sodium phosphate buffer at pH 7.

### Surface modification of the gold colloids: preparation of the glucose aggregate nanosensors

The immobilization of dextran on gold nanoparticles was performed using the following four steps: (A) chemisorption of a long-chain carboxyl-terminated alkane thiol on gold nanoparticles as described previously [1]; (B) the activation of surface carboxyl groups using EDC and NHS; (C) activation of hydroxyl groups using epicholorohydrin; and (D) the covalent coupling of dextran.

Gold nanoparticle dispersions with a concentration of 0.80nM for 20nm, and 8nM for 10 nm gold colloids (determined by measuring absorbance at 520nm and using extinction coefficients of 1.25 × 10⁹ and 1.21 × 10⁸ M⁻¹cm⁻¹ for 20 and 10 nm gold, respectively; Sigma) were degassed with nitrogen before use. The gold colloids are not naked, but indeed solution stabilized with the citrate counter ion. The addition of the alkane thiol (step 1) replaces the citrate counter ion, producing a stabilized monolayered protected particle [1]. Equal volumes (400*u*l) of gold nanoparticle dispersions (0.80/8nM, before mixing) and Tween 20 (1.82mg/ml, before mixing) in pH 7 buffer were gently mixed and allowed to stand for 30min for the physisorption of the Tween 20 to the gold nanoparticles [1]. Four hundred microliters of 0.50mM 16-MHDA was then added and the final mixture (final concentrations: [gold nanoparticles] = 0.27/2.67nM; [Tween 20] = 0.61 mg/ml; [16-MHDA] = 0.17 mM) was allowed to stand for 3h for the chemisorption of 16-MHDA to be completed on the gold colloids, while simultaneously displacing Tween 20 [1]. The time requirement for chemisorption of both the Tween and subsequent displacement with MHDA can be reduced by using low-power microwave energy.

In order to remove excess 16-MHDA and Tween 20, the final mixture was centrifuged (three times for 15 min at 16 060 × *g*; the supernatants were discarded after each cycle) and resuspended in phosphate buffer (with 1.82 mg/ml Tween 20 at pH 7). 16-MHDA-modified gold colloids that remained in the centrifugate were then reacted with a mixture of freshly prepared 50 mM NHS and 200 mM EDC solution (in phosphate buffer without Tween 20) for 5min. The resulting nanoparticle dispersion was centrifuged (5 min, 16 060 × g) and after discarding the supernatant, the remaining NHS ester-alkane thiol-modified gold nanoparticles were reacted with a freshly prepared solution of AEE (2%, v/v) for 10 min. Excess AEE was removed by centrifugation (for 5 min at 16 060 × *g* at least three times). The retentate that contained AEE-modified gold nanoparticles was centrifuged (5min, 16 060 × g). The hydroxyl groups on the AEE-modified gold nanoparticles were activated with 0.6 M epicholorohydrin solution in a 1:1 mixture of 0.4 M NaOH and diglyme for 4 h at room temperature. The nanoparticle dispersion was then centrifuged for 10 min at 16 060 × g and resuspended in diglyme and centrifuged again to remove the excess epicholorohydrin. The centrifugate, containing AEE-modified gold nanoparticles with active epoxide groups, were incubated in dextran solution (0.1 M NaOH) for 20 h [2]. Again, the time requirement for attachment of dextran to the AEE-modified gold nanoparticles can be reduced by using low-power microwave energy.

Finally, dextran-modified gold nanoparticles were centrifuged for 15 min at 16 060 ×g and resuspended in 0.1 M NaOH and centrifuged four more times to remove the excess dextran. All solutions of dextran-coated gold nanoparticles were stored in polypropylene centrifuge tubes in the dark to prevent light-induced flocculation of the nanoparticles and oxidation of the alkane thiols [3].

To monitor the extent of aggregation, which clearly showed a significant change in absorbance, Δ*A*₆₅₀, between a highly aggregated system and a slightly aggregated system. The time required to complete aggregation for the same set of samples was additionally investigated by monitoring the Δ*A*₆₅₀ as a function of time. As expected, samples with greater additions of Con A showed shorter 90% absorbance (of the final absorbance maximum value) change times, simply reflecting a quicker aggregation rate. Aggregation time can be reduced by applying low-power microwave energy thereby providing usable nanoparticles in a shorter time-period without damaging any of the components.

### EXAMPLE 2

This example illustrates a microwave accelerated plasmonics application in which low power microwaves are utilized in solution based plasmon resonance particle assays.

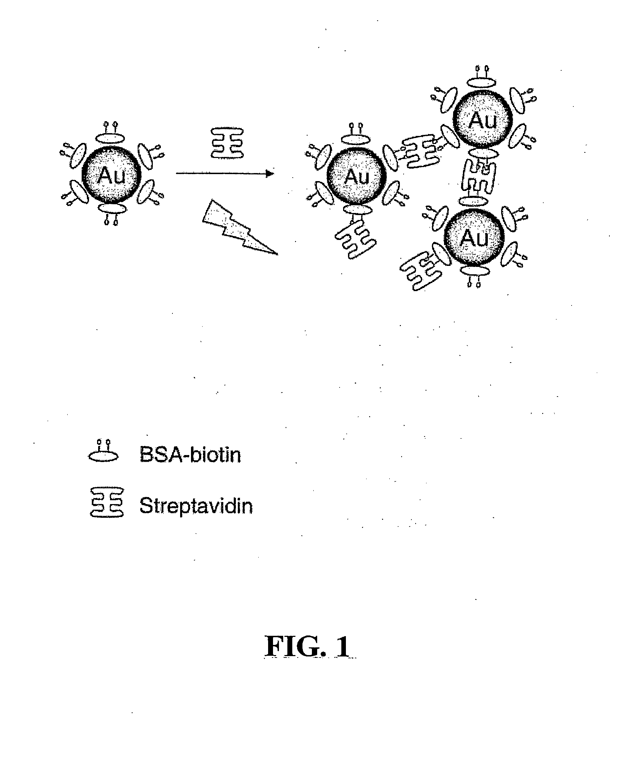

As a model protein based system, biotinylated-bovine serum albumin coated 20 nm gold particles, crosslinked by additions of streptavidin (a tetravalent protein), are employed. The model system is schematically shown in FIG. 1, in an illustrative depiction of the gold particles (Au) coated with BSA-biotin, undergoing aggregation in the presence of streptavidin and low level microwave energy input.

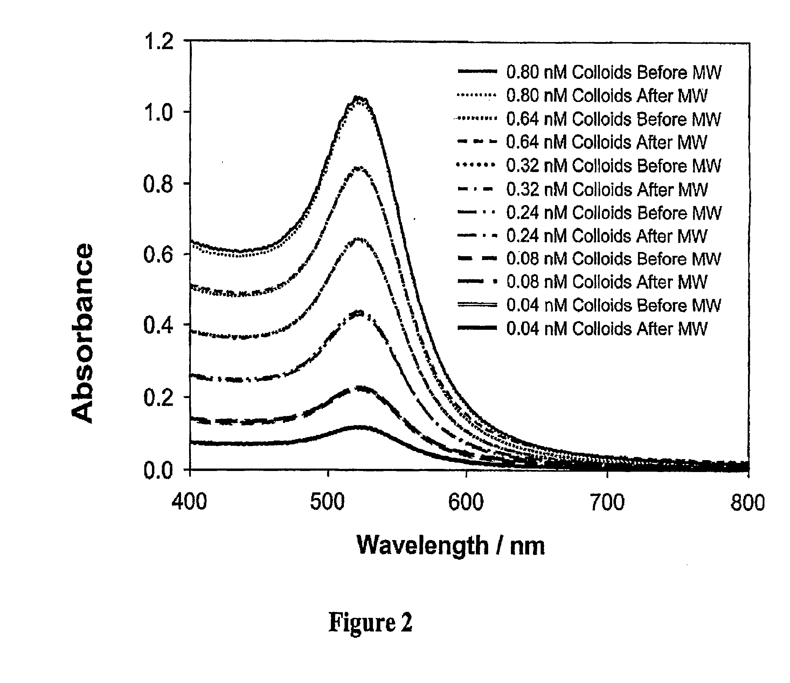

FIG. 2 shows absorption spectra of 20 nm gold colloids as a function of concentration, before and after exposure to low power microwaves. In Figure 2, we can see a range of different concentrations of 20 nm gold colloids (0.80nM; 0.64nM; 0.32nM; 0.24nM; 0.08nM; and 0.04nM) . The plasmon resonances are not perturbed by the low power heating, as shown by the close congruence of the respective curves before and after microwave (MW) exposure, at each of the concentrations over the range, evidencing the surprising and unexpected character of the discovery of the present invention.

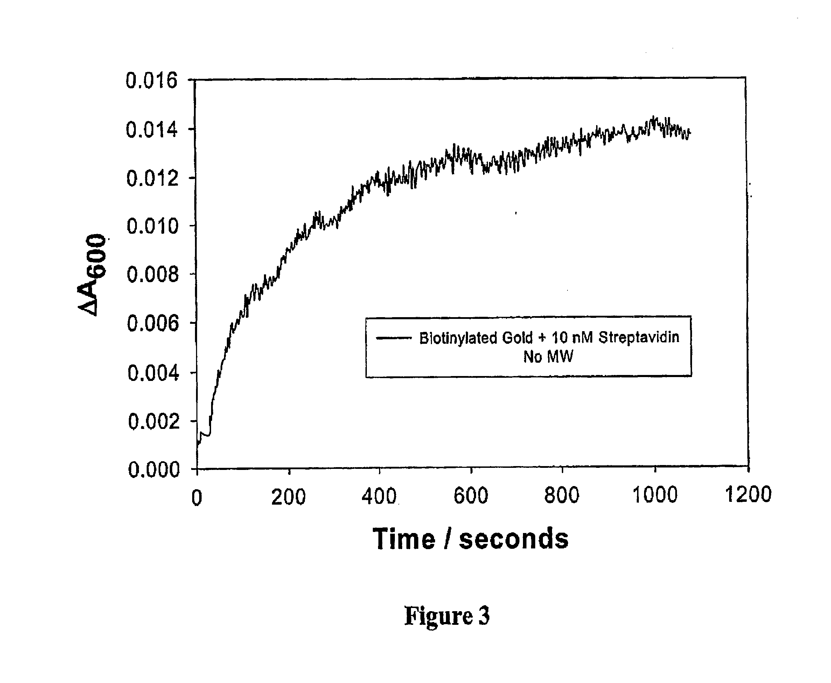

Upon aggregation addition of 10 nM of streptavidin to the particles as schematically shown in FIG. 1, the particles rapidly aggregate. By monitoring the change in absorbance at an arbitrary wavelength (red-shifted from the Abs plasmon maxima), an increase in the absorbance at 600 nm is observed. This is shown in FIG. 3, which is a graph of the change in absorbance at 600 nm as a function of time for the biotinylated-BSA coated 20 nm gold colloids crosslinked by streptavidin. This figure indicates that the room temperature reaction is > 90% kinetically complete at about 900 seconds, the graph indicating that the reaction likely requires a reaction time in excess of 20 minutes (1200 secs) to be kinetically complete.

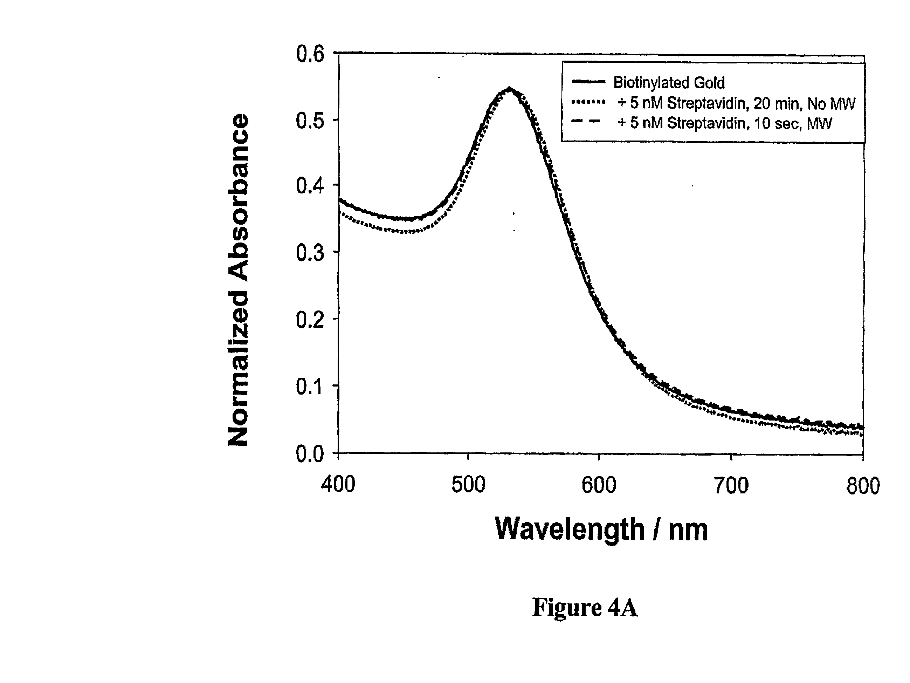

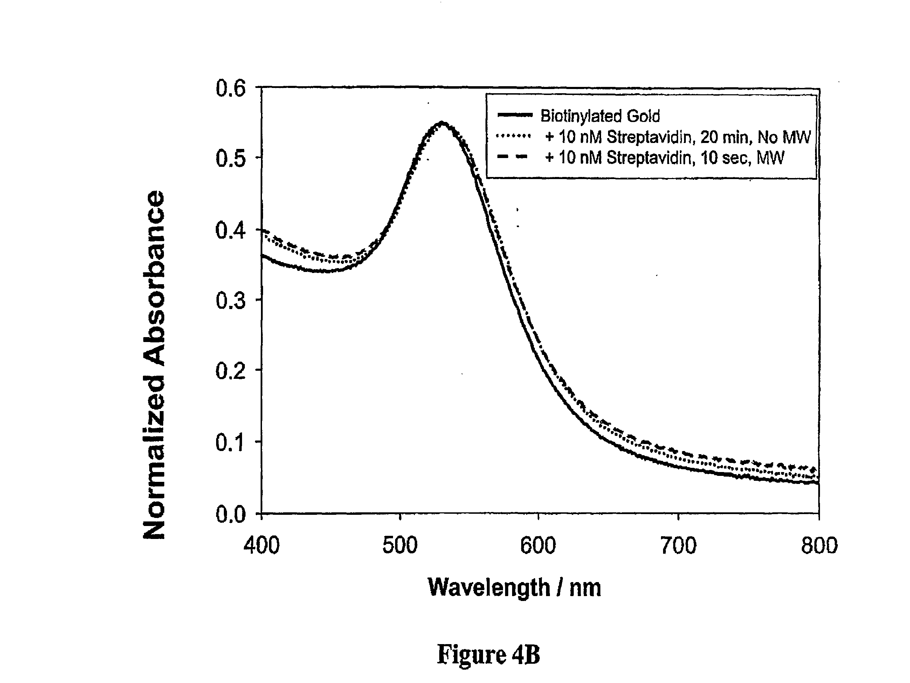

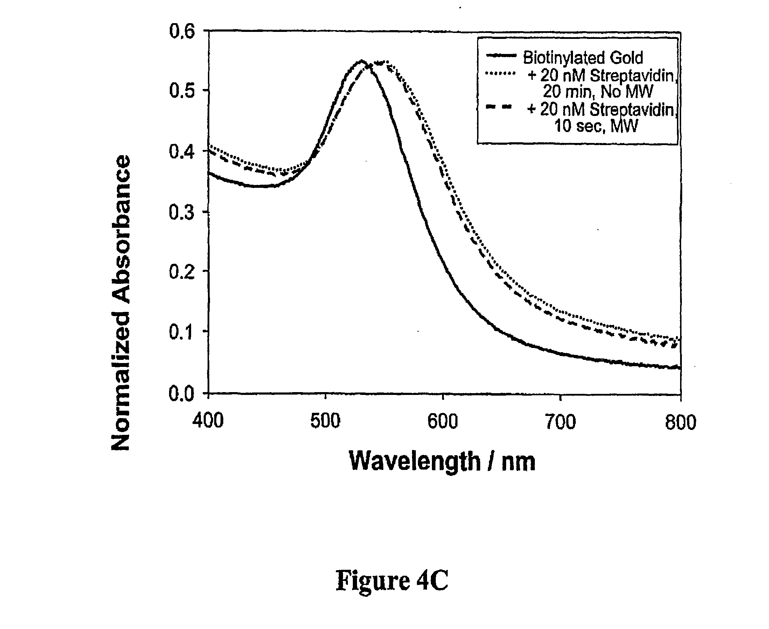

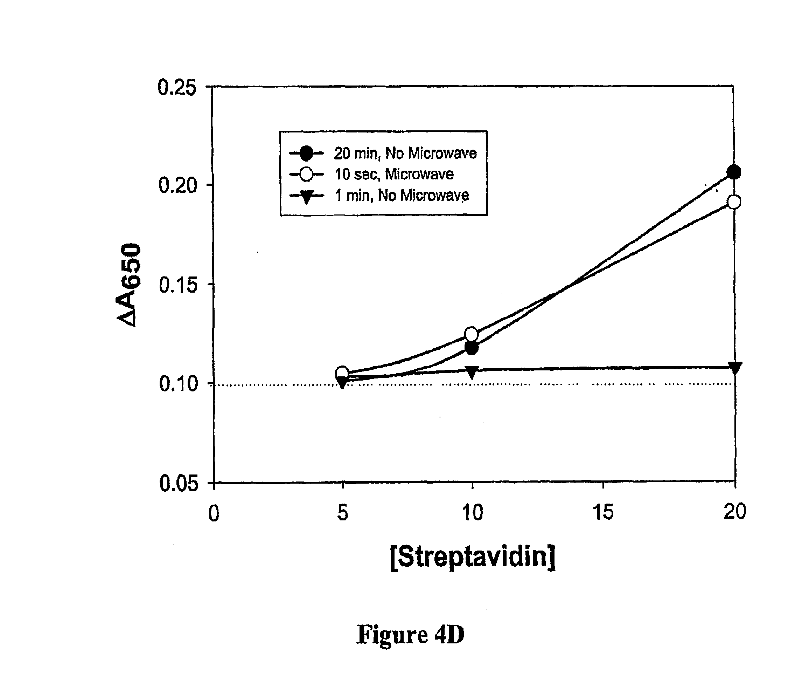

By contrast, when the identical assay is heated using low power microwaves, the reaction is essentially complete in about 10 seconds, as is apparent from FIG. 4.

FIG. 4 shows the change in absorbance of biotinylated-BSA 20 nm gold colloids crosslinked by different additions of streptavidin, both without (20 min incubation) and after low power microwave heating, with FIG. 4A showing the effect of a 5 nM streptavidin addition, FIG. 4B showing the effect of a 10 nM streptavidin addition, FIG. 4C showing the effect of a 20 nM streptavidin addition, and FIG. 4D showing the change in absorbance at 650 nm for both the room temperature incubated and microwave heated samples.

These results shown that using low power microwaves to kinetically accelerate the assay affords for a greater than 90-fold increase in assay kinetics (10 seconds versus 900 seconds). For rapid diagnostic tests, such as for example are desired in clinical or bio-terrorism related applications, in instances in which a clinician or first responder needs to make an informed assessment very quickly, the invention therefore evidences the capability for significantly facilitating assay rapidity and alleviating current issues and bottlenecks in respect of assay run time.

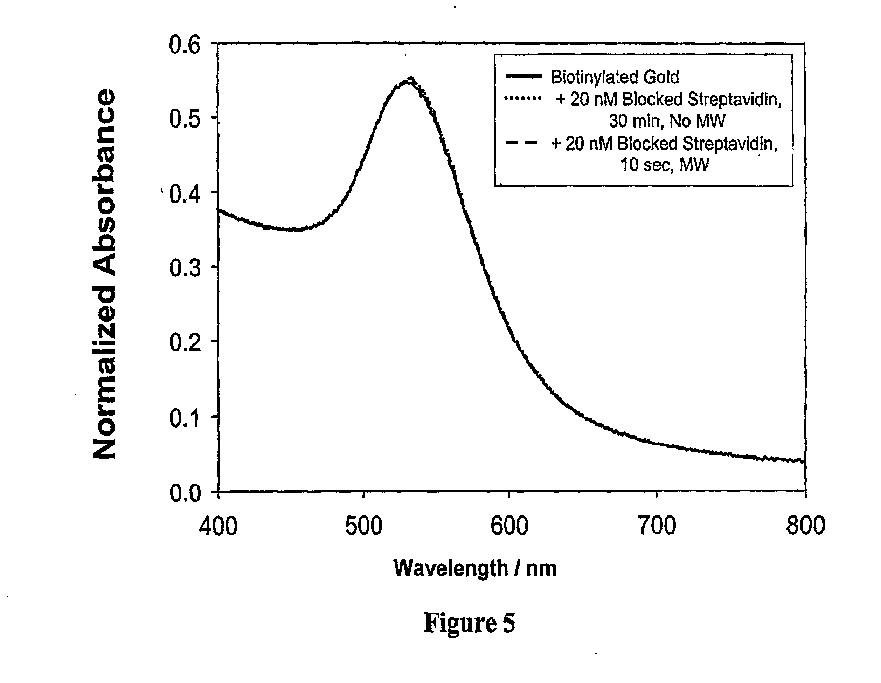

The extent of non-specific absorption upon low power microwave heating, using blocked streptavidin, was also investigated, with the results shown in FIG. 5.

FIG. 5 shows absorption spectra of biotinylated-BSA 20 nm gold colloids in the presence of blocked streptavidin, after both a 30 minute incubation period and 10 seconds microwave heating. The low power microwaves did not facilitate any non-specific absorption by the Biotinylated-BSA coated colloids.

In application to long-term (extended life) sensor implementations, or long-term usage reversible sensors, it is important for the colloids or other plasmon resonance based structures to be unperturbed by heating. To assess the suitability of the microwave-accelerated plasmonics process and systems of the invention for such applications, solutions of colloids were heated to temperatures between 20°C and 80°C. In all examples, both 20 and 200 nn colloids had plasmon absorption spectra, which were substantially identical before and after heating.

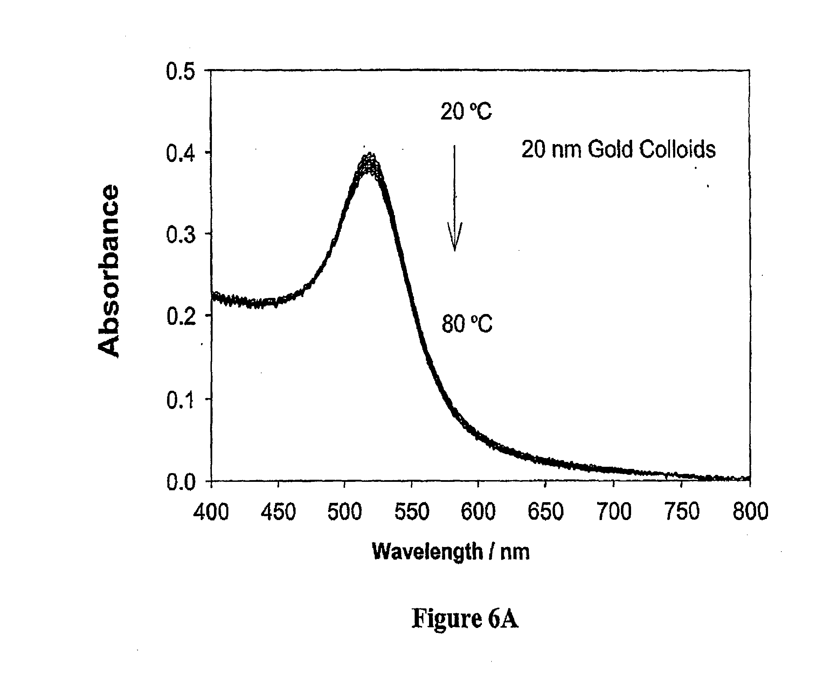

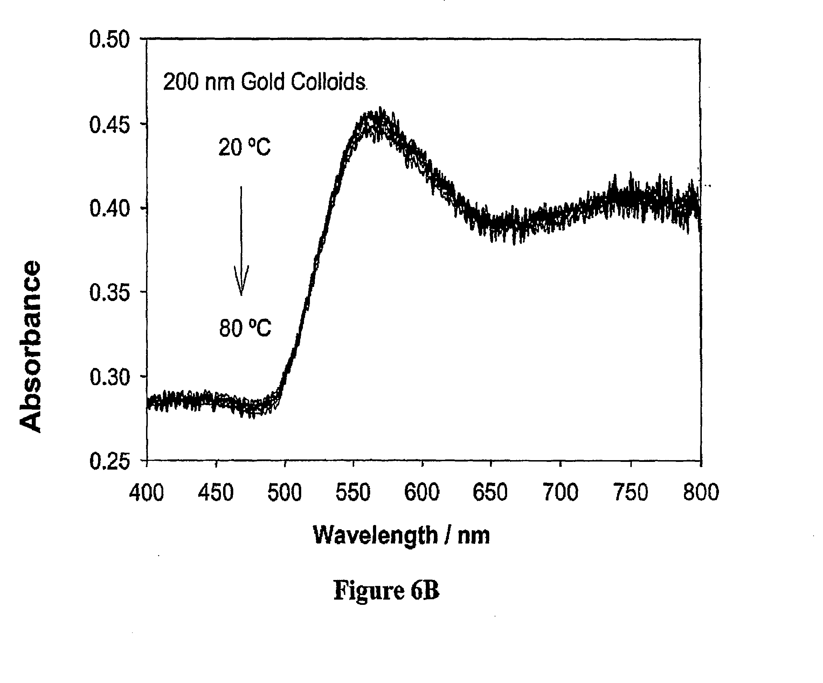

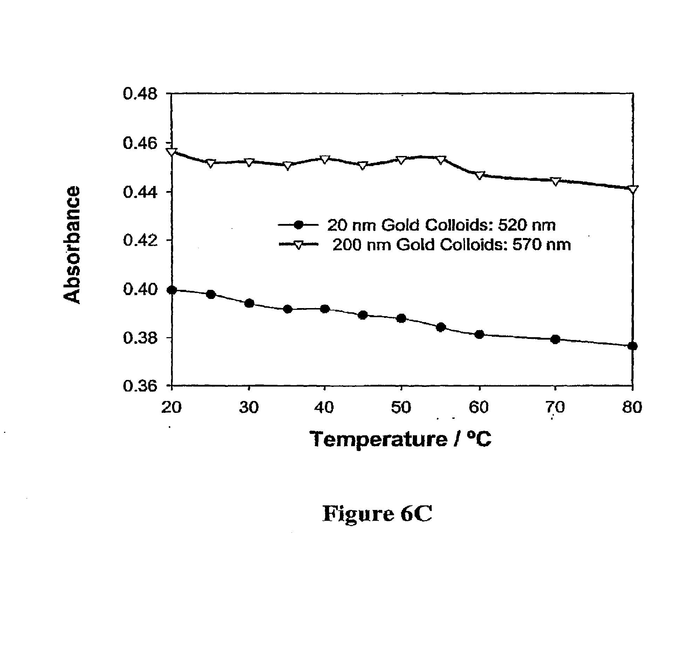

FIG. 6 shows the change in absorbance of both 20nm (FIG. 6A) and 200 nm (FIG. 6B) virgin gold colloids as a function of temperature, and the respective change in absorbance as a function of temperature (FIG. 6C).

FIG. 6C shows that the total plasmon absorption maxima at either 520 nm (for 20 nm colloids) or 570 nm (for 200 nm colloids) is slightly reduced at elevated temperatures, which is potentially attributable to the difference in refractive index of water (buffered media) at 20°C as compared to 80°C.

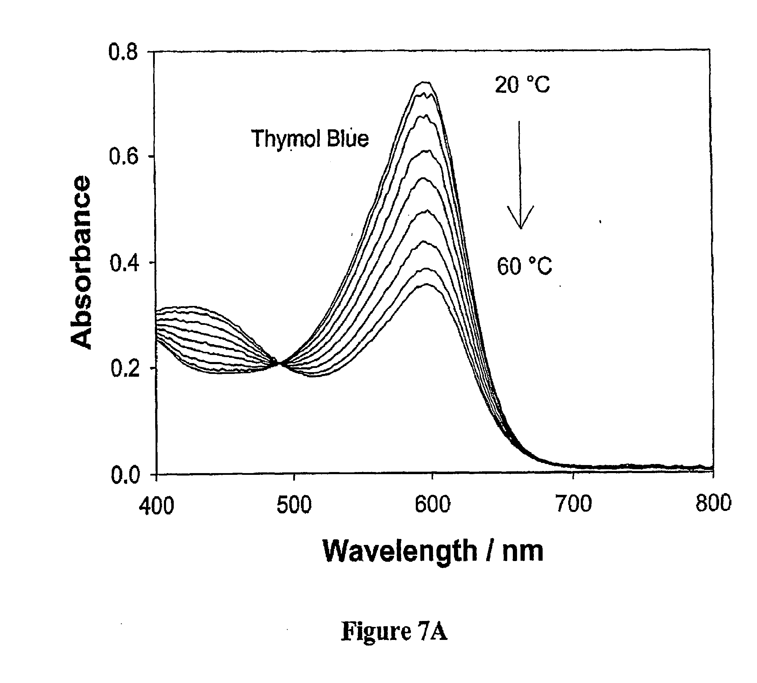

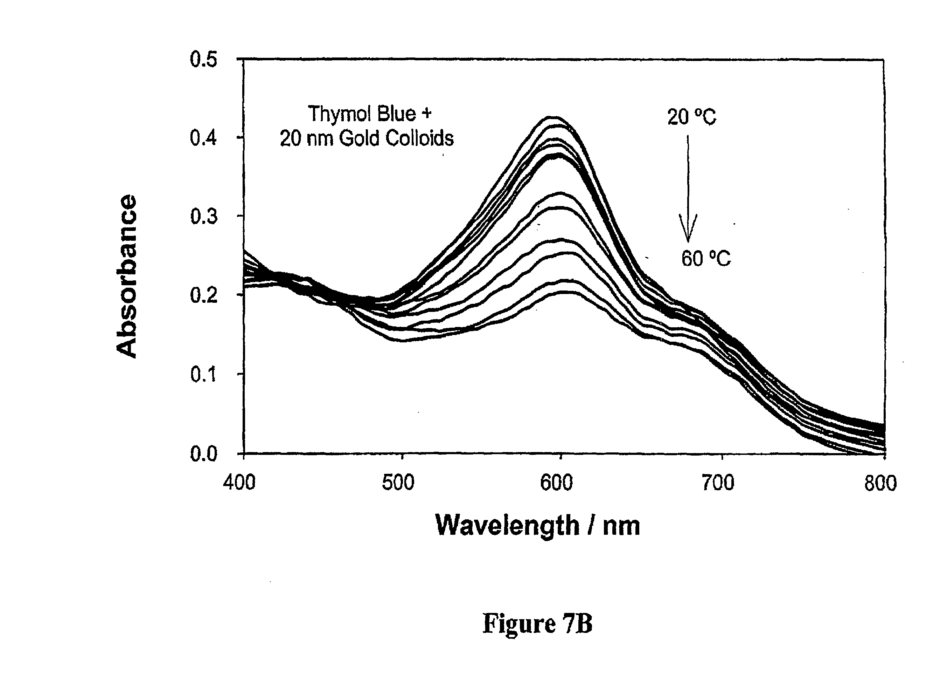

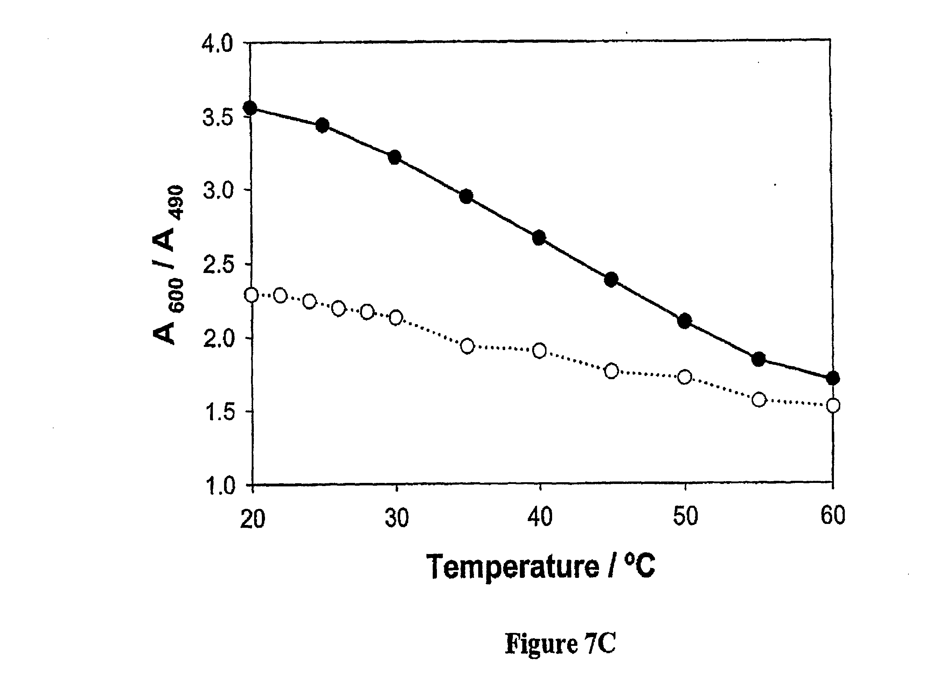

In order to ascertain the temperature jump in the low power microwave heated colloidal system that facilitates the observed > 90-fold increase in assay kinetics, a temperature dependent probe, thymol blue (0.5 mM thymol blue, in 50 mM tris acetate, pH 9.0) was utilized. The absorption spectrum of thymol blue was recorded as the temperature was gradually increased from 20°C to 80°C. The results are shown in FIG. 7, which includes an absorption spectrum of thymol blue as a function of temperature (FIG.7A), the same solution containing 20 nm colloids (FIG. 7B), and the respective A600 / A425 ratiometric plots as a function of temperature (FIG. 7C).

The color of the solution changed with temperature (not microwave heated) from deep magenta to pale yellow, due to the temperature dependence of the ionization constant of the tris buffer. As the temperature was increased, the pH of the solution decreased and the distribution of the ionization states of the thymol blue dye changed, resulting in a color change as a function of temperature. The reversible color change was readily observed in the UV-Vis spectrum by changes in the 425 nm and 600 nm spectral bands. In addition, the change in absorption spectrum was determined as 20 nm gold colloids were heated (not microwave heated) in the presence of the thymol blue. A shoulder developed at ∼ 680 nm, which is due to the high pH of the tris buffer.

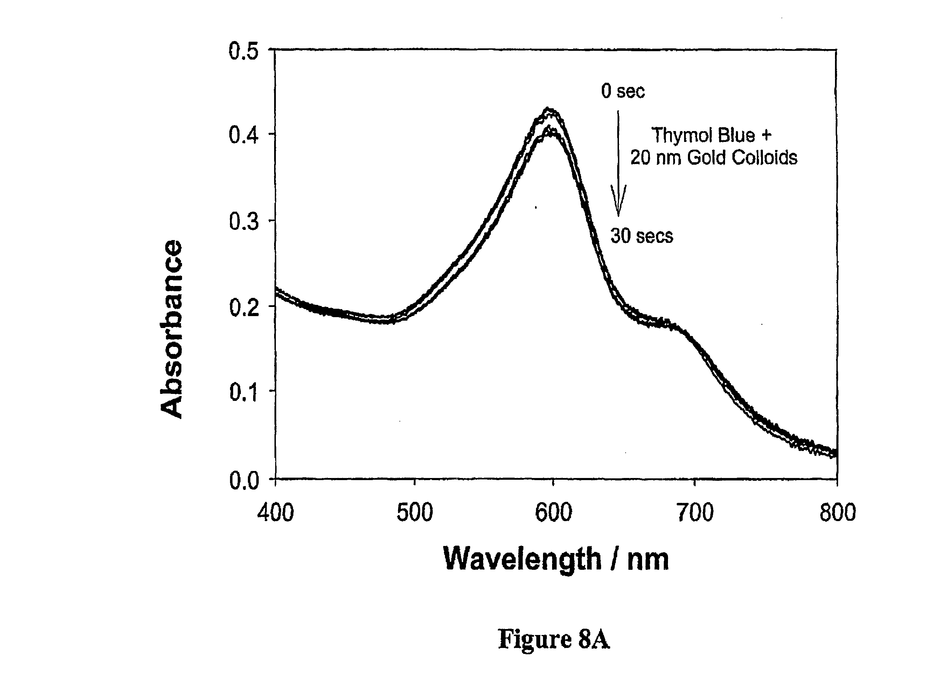

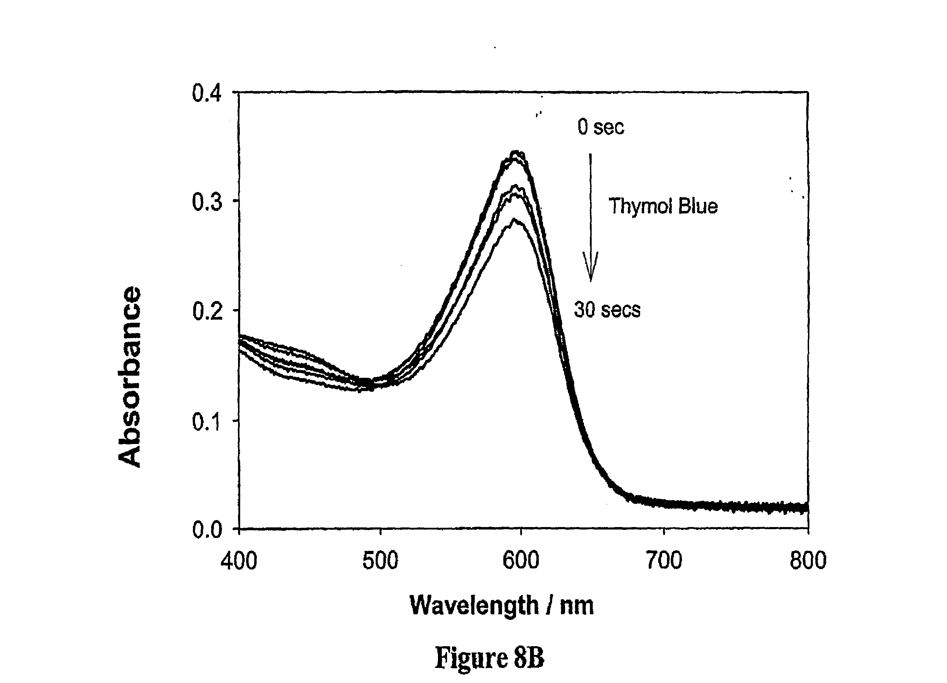

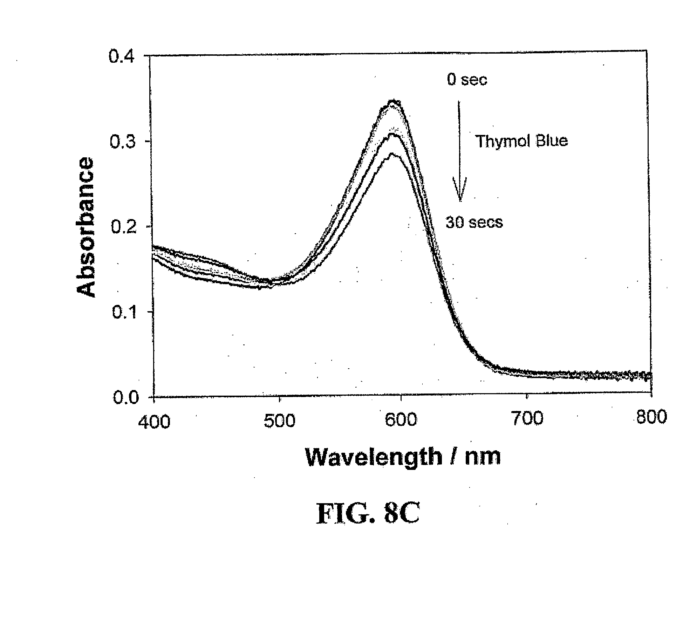

FIG. 8 shows absorbance spectra of 450 µL of 20 nm gold colloids and thymol blue microwave heated in the black body for different times (FIG. 8A), 450 µL of thymol blue solely heated in the black body (FIG. 8B), and the respective ratiometric plots (FIG. 8C).

When a 0.27 nm solution of gold colloids and thymol blue was microwave heated (FIG. 8A), it can be deduced that the temperature jump of the bulk solvent is approximately 5°C for 10 seconds heating (FIG. 8C). Given the > 90-fold change in assay kinetics shown in FIG. 4, this suggests that the local temperature around/of the colloids is significantly higher, since a 5°C temperature jump of the local solvent does not explain the observed 90-fold increase in assay kinetics. Preferential heating of plasmon resonance particles in the microwave cavity, relative to the bulk solvent, thus emerges as a causal factor to account for the rapid kinetics observed. Such local heating of the colloids enables increased assay kinetics (temperature accelerated kinetics) to occur close to the particles, and promotes greater particle momentum in solution, so that the particles are able to sense a greater solution volume per unit time.

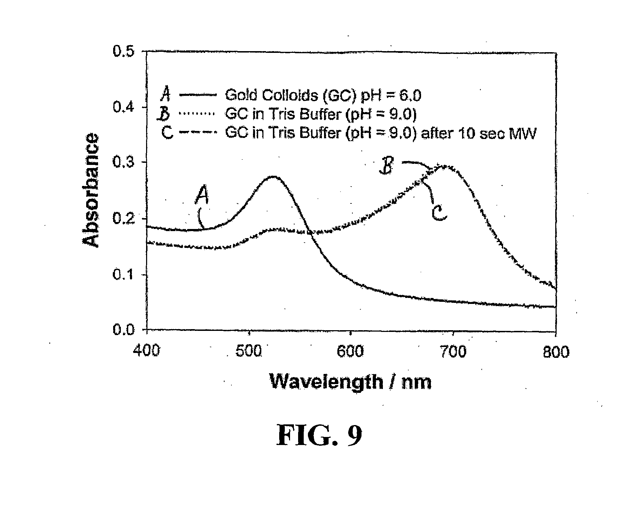

FIG. 9 shows absorbance spectra of 20 nm gold colloids in pH 6.0, 9.0 and after microwave heating.

### REFERENCES

The contents of all references are hereby incorporated by reference herein for all purposes.
1. K. Aslan, V.H, Perez-Luna, Langmuir 18 (2002) 6059-6065.
2. S. Lofas, B. Johnson, J. Chem. Soc. Chem. Commun. (1990) 1526.
3. C.S. Weisbecker, M.G. Merritt, G. M Whitesides, Langmuir 12 (1996) 3763-3772.

Although the invention has been described with respect to specific embodiments, the details are not to be construed as limitations.

## Claims

1. A detection method comprising:
providing metallic nanoparticles wherein the metallic nanoparticles are modified with multiple immobilized groups having affinity for a complexing agent;
introducing the complexing agent for binding with immobilized groups;
applying microwave energy having a frequency range of from 10⁸ to 10¹² Hz to cause an increase in heat in the system and/or increase the kinetics of a chemical reaction between the immobilized group and complexing agent within the detection system thereby increasing aggregation of the metallic particles when the complexing agent and immobilized group react, wherein the microwave energy has an intensity in a range of from about 0.0001 µW/cm² to about 1000 µW/cm²; and
measuring the change in plasmonic absorption due to aggregation.

2. The detection method of claim 1, wherein the metallic nanostructure comprises gold or silver nanoparticles.

3. The detection method of claim 2, wherein dextran is the group immobilized on the metallic nanostructures.

4. The detection method of claim 3, wherein the complexing agent comprises Concanavalin A.

5. The detection method of claim 1, further comprising detecting the presence or concentration of the complexing agent by a change in plasmonic resonance of the particles, wherein said change comprises a change in the scattering properties of said particles.

6. The detection method of claim 1, further comprising detecting the presence or concentration of the complexing agent by a change in plasmonic resonance of the particles, wherein said change comprises a colorimetric change.

7. The detection method of claim 1, wherein said microwave energy is non-degradative of the target and sample.

8. The detection method of claim 1, as adapted for glucose monitoring.

9. The detection method of claim 4, wherein the complexing agent has affinity for dextran and glucose.

10. The method according to claim 9, wherein the dextran is complexed with thiolate boronic acid.

## Patentansprüche

1. Nachweisverfahren, das Folgendes umfasst:
Bereitstellen von metallischen Nanopartikeln, wobei die metallischen Nanopartikel durch mehrere immobilisierte Gruppen mit einer Affinität für einen Komplexbildner abgeändert sind;
Einführen des Komplexbildners zur Bindung mit immobilisierten Gruppen;
Anwenden von Mikrowellenenergie mit einem Frequenzbereich von 10⁸ bis 10¹² Hz zum Verursachen eines Anstiegs der Wärme im System und / oder eines Anstiegs der Kinetik einer chemischen Reaktion zwischen der immobilisierten Gruppe und dem Komplexbildner innerhalb des Nachweissystems und dadurch Erhöhung der Ansammlung der metallischen Partikel bei Reaktion des Komplexbildners und der immobilisierten Gruppe, wobei die Mikrowellenenergie eine Intensität zwischen ca. 0,0001 µW / cm² und ca. 1000 µW / cm² aufweist; und
Messen der Änderung der Plasmonenabsorption aufgrund der Ansammlung.

2. Nachweisverfahren nach Anspruch 1, wobei die metallische Nanostruktur Nanopartikel aus Gold oder Silber umfasst.

3. Nachweisverfahren nach Anspruch 2, wobei Dextran die auf den metallischen Nanostrukturen immobilisierte Gruppe ist.

4. Nachweisverfahren nach Anspruch 3, wobei der Komplexbildner Concanavalin A umfasst.

5. Nachweisverfahren nach Anspruch 1, weiter umfassend den Nachweis des Vorhandenseins oder der Konzentration des Komplexbildners durch Änderung der Plasmonenresonanz der Partikel, wobei die besagte Änderung eine Änderung der Streuungseigenschaften der besagten Partikel umfasst.

6. Nachweisverfahren nach Anspruch 1, weiter umfassend den Nachweis des Vorhandenseins oder der Konzentration des Komplexbildners durch Änderung der Plasmonenresonanz der Partikel, wobei die besagte Änderung eine kolorimetrische Änderung umfasst.

7. Nachweisverfahren nach Anspruch 1, wobei die besagte Mikrowellenenergie in Bezug auf das Ziel und das Muster nicht degradierend ist.

8. Nachweisverfahren nach Anspruch 1, wie für die Glucose-Messung angepasst.

9. Nachweisverfahren nach Anspruch 4, wobei der Komplexbildner eine Affinität für Dextran und Glucose aufweist.

10. Verfahren nach Anspruch 9, wobei das Dextran mit Thiolat-Boronsäure komplexiert ist.

## Revendications

1. Une méthode de détection comprenant :
la fourniture de nanoparticules métalliques, les nanoparticules métalliques étant modifiées avec de multiples groupes immobilisés présentant une affinité pour un agent complexant ;
l'introduction de l'agent complexant pour la liaison avec des groupes immobilisés ;
l'application d'une énergie de micro-ondes ayant une plage de fréquences comprises entre 10⁸ et 10¹² Hz pour donner lieu à une augmentation de la chaleur dans le système et/ou une augmentation de la cinétique d'une réaction chimique entre le groupe immobilisé et l'agent complexant au sein du système de détection, en augmentant ainsi l'agrégation des particules métalliques lors de la réaction entre l'agent complexant et le groupe immobilisé, l'énergie des micro-ondes présentant une intensité comprise dans une page d'environ 0,0001 µW/cm² à environ 1 000 µW/cm²; et
la mesure de la variation de l'absorption plasmonique due à l'agrégation.

2. La méthode de détection selon la revendication 1, dans laquelle la nanostructure métallique comprend des nanoparticules d'or ou d'argent.

3. La méthode de détection selon la revendication 2, dans laquelle dextran est le groupe immobilisé sur les nanostructures métalliques.

4. La méthode de détection selon la revendication 3, dans laquelle l'agent complexant comprend de la concanavaline A.

5. La méthode de détection selon la revendication 1, comprenant en outre la détection de la présence ou de la concentration de l'agent complexant par une variation de résonance plasmonique des particules, ladite variation comprenant une variation des propriétés de diffusion desdites particules.

6. La méthode de détection selon la revendication 1, comprenant en outre la détection de la présence ou de la concentration de l'agent complexant par une variation de résonance plasmonique des particules, ladite variation comprenant une variation colorimétrique.

7. La méthode de détection selon la revendication 1, ladite énergie de micro-ondes n'engendrant aucune dégradation de la cible et de l'échantillon.

8. La méthode de détection selon la revendication 1, étant adaptée pour le contrôle du glucose.

9. La méthode de détection selon la revendication 4, l'agent complexant présentant une affinité pour le dextran et le glucose.

10. La méthode selon la revendication 9, le dextran étant complexé avec de l'acide boronique thiolate.
